# EUROPÄISCHE PATENTSCHRIFT

(11) **EP 1 656 972 B1**
(45) Veröffentlichungstag und Bekanntmachung des Hinweises auf die Patenterteilung: **09.09.2009**
(21) Anmeldenummer: 05023574.6
(22) Anmeldetag: 28.10.2005
(51) Int. Cl.: A63B 23/02, A63B 21/055, A61F 5/02, A47C 7/42

(54) **Gerät zum Trainieren der Bauchmuskel in sitzender Position**
Device for exercising the abdominal muscles in sitting position
Appareil pour exercer les muscles abdominaux en position assise

(30) Priorität: 14.11.2004 DE 102004055235
(43) Veröffentlichungstag der Anmeldung: 17.05.2006
(73) Patentinhaber: Fa. SÖZ, 90427 Nürnberg (DE)
(72) Erfinder:
(74) Vertreter: Küchler, Stefan

(56) Entgegenhaltungen:
- US-A- 5 141 482
- US-A- 5 205 814
- US-A- 6 063 012
- US-B1- 6 500 104

## Beschreibung

Die Erfindung richtet sich auf ein Gerät zum Trainieren der Bauchmuskel in sitzender Position, mit einem Gurt, der vom Anwender durch aktive Muskelanspannung bewegt wird, und mit einer Einrichtung zur vorzugsweise lösbaren Festlegung des Gurtes an der Rückenlehne eines Stuhls, Autositzes, oder eines sonstigen Sitzmöbels, mit den gattungsgemäßen Merkmalen des Anspruchs 1.

Aus der DE-OS 100 06 588 A1 ist ein Gürtel zur Haltungsverbesserung und zum Bauchmuskeltraining bekannt, der um die Taille getragen wird und einen Zugkraftsensor aufweist, der bei einer zu starken Entspannung der Bauchmuskulatur einen Vibrationsalarm auslöst, um den Träger des Gürtels an die Kontraktion der Bauchmuskulatur zu erinnern.

Dieser Gürtel wird von der betreffenden Person bevorzugt unter einem Kleidungsstück getragen und sollte daher immerfort angelegt bleiben. Er ist für diesen speziellen Anwendungsfall optimiert und kann nicht für das Training anderer Muskeln verwendet werden, insbesondere auch deshalb, weil nur ein vergleichsweise kurzes Gürtelsegment überhaupt elastisch ist und daher eine größere Dehnung nicht möglich ist.

Ferner fehlt in unbenutztem Zustand eine Möglichkeit zur definierten Verwahrung an einem bestimmten Ort, wo der Gürtel schnell wiedergefunden werden könnte.

Das US-Patent 5,669,671 betrifft einen Stützgurt für eine auf einem Stuhl sitzende Person. Dabei wird ein erster Gurt um die Sitzlehne geschlungen und mittels einer Schnalle in sich geschlossen. Auf diesem Gurt sitzen zwei Befestigungselemente, mit je einer Öse. In diese Ösen wird je ein Haken eingehängt, der an einem Ende eines kurzen Beckengurtabsschnittes festgelegt ist. An den freien Enden dieser beiden Beckengurtabsschnitte befindet sich je ein Teil eines Gurtschlosses, mit dem die freien Enden der beiden Beckengurtabschnitte miteinander verbunden werden können. Mit einer solchen Einrichtung ist es einer Person möglich, sich an einem beliebigen Stuhl anzugurten, um die Körpermuskulatur während langer Sitzphasen zu entlasten. Ein Bauchmuskeltraining ist mit dieser Einrichtung nicht vorgesehen. Dazu ist auch die Festlegung dieser Einrichtung an einem Stuhl mittels des um die Rückenlehne zu schlingenden Gurtes überhaupt nicht geeignet: Dieser Befestigungsgurt könnte entweder elastisch ausgeführt werden oder unelastisch. Im ersteren Fall würde er bei einer Anspannung der Bauchmuskulatur ständig gedehnt werden und daher gegenüber der Sitzlehne scheuern und diese allmählich beschädigen; in letzterem Fall könnte er nicht straff um die Rückenlehne gespannt werden und wird daher unkontrolliert verrutschen, so dass eine definierte Übungsposition nicht sichergestellt ist.

Bei dem US-Patent 5,141,482 ist ein Brustgeschirr vorgesehen, sowie ein um eine Sitzlehne zu schlingender Befestigungsgurt; dazwischen verlaufen zwei elastische Bänder, welche durch rhythmisches Bewegen des Oberkörpers nach vorne und nach hinten angespannt und wieder entspannt werden können. Hierzu ist jedoch ein ständiges Verlagern des Oberkörpers erforderlich, was während einer Tätigkeit, insbesondere jedoch während einer Autofahrt, hinderlich oder gefährlich, ja sogar lebensgefährlich sein kann.

Ähnliches gilt für die Anordnung nach dem US-Patent 6,063,012: Hier erstrecken sich zwei ein Bänder um den Oberkörper einer auf einem Stuhl sitzenden Person und sind hinter der Lehne des betreffenden Stuhls miteinander verbunden, vorzugsweise durch eine elastische Schlaufe. Dabei muß die betreffende Person jedoch eine Platte vor der Brust halten, an welcher die Enden der beiden Bänder fixiert werden. Eine solche Brustplatte ist jedoch für die meisten Tätigkeiten wie auch für eine Autofahrt hinderlich oder gar gefährlich.

Das US-Patent 6,500,104 beschreibt ein Übungsgerät, bestehend aus einer flachen Sitzeinheit mit einem Sitz- und einem Rückenkissen. An den Seitenrändern dieser Sitzeinheit sind Ösen angeformt, worin Expander eingehängt werden können, welche mit der Kraft der Arme gedehnt werden müssen. Damit läßt sich evtl. die Armmuskulatur trainieren, jedoch nicht die Bauchmuskulatur.

Schließlich offenbart das US-Patent 5,205,814 ein Lordosekissen, das mittels eines Gurtes am Körper befestigt wird und beim Sitzen auf einem Stuhl od. dgl. Die Lendenwirbelsäule unterstützt und dadurch einer ungesunden Verformung vorbeugt. Ein Bauchmuskeltraining ist damit nicht möglich, u.a. auch deshalb, weil der Gurt zur Befestigung des Lordosekissens am Körper nicht elastisch ausgebildet ist.

Aus den Nachteilen des geschilderten Standes der Technik resultiert daher das die Erfindung initiierende Problem, ein gattungsgemäßes Gerät zum Trainieren der Bauchmuskel in sitzender Position derart weiterzubilden, dass der Zugkraft-Gurt jederzeit schnell abgelegt werden kann und dennoch stets einsatzbereit ist oder evtl. gar zum Training anderer Muskel verwendet werden kann, wobei das Gerät in an einem Stuhl festgelegtem Zustand Übungen der Bauchmuskulatur erlauben soll, ohne dass dabei eine ständige Bewegung des Oberkörpers erforderlich ist, so dass während der Übungen auch andere Tätigkeiten ausgeführt werden können oder bspw. Ein Fahrzeug gesteuert werden kann.

Zur Lösung dieses Problems sieht die Erfindung ein Gerät zum Trainieren der Bauchmuskel in sitzender Position vor, mit einem Gurt, der vom Anwender durch aktive Muskelanspannung bewegt wird, mit einer Einrichtung zur vorzugsweise lösbaren Festlegung des Gurtes an der Rückenlehne eines Stuhls, Autositzes, oder einem sonstigen Sitzmöbel, vor, und mit den weiteren Merkmalen des Anspruchs 1.

Gerade beim bequemen Sitzen im Auto oder Büro ist man allzu leicht geneigt, die Bauchmuskulatur völlig zu entspannen, so dass diese allmählich erschlafft. Deshalb ist ein Zugkraft-Gurt zum Bauchmuskeltraining gerade beim Sitzen besonders wichtig, während die Bauchmuskel beim Gehen oder Stehen ohnehin ständig angespannt sind, um das Gleichgewicht zu halten. Indem der Gurt daher an einem Stuhl oder Sitz befestigbar ist, kann er beim Platznehmen stets angelegt werden und steht sodann für das Bauchmuskeltraining zur Verfügung. Dies ist gerade beim Autofahren vorteilhaft, wo während längerer Fahrten keine andere Bewegungsmöglichkeit besteht und durch das Bauchmuskeltraining auch der Kreislauf in Schwung gehalten werden kann. Der Zugkraft-Gurt selbst umschließt die Taille einer Person nur teilweise, vorzugsweise auf höchstens 270° oder weniger; der verbleibende Taillenumfang wird von der Befestigungseinrichtung umschlossen, die an der Rückenlehne des betreffenden Stuhls oder Sitzes festgelegt ist. An einem oder vorzugsweise beiden Enden des Zugkraft-Gurtes ist je ein Verbindungsmittel angeordnet, bspw. ein Steckverbindungselement. Damit kann der Zugkraft-Gurt teilweise oder vollständig von der Befestigungseinrichtung gelöst werden. Wird nur ein Ende gelöst, so verbleibt der Zugkraft-Gurt einsatzbereit an dem betreffenden Sitz, die Person kann jedoch aufstehen und ihren Platz verlassen. Werden beide Enden gelöst, so kann der Zugkraft-Gurt von dem Sitz gelöst und anderweitig verwendet werden, bspw. nach Art eines Expanders zum Trainieren der Armmuskulatur od. dgl. Da der Befestigungs-Gurt in begrenztem Umfang elastisch ausgebildet ist, so zieht er sich um die Rückenlehne zusammen und liegt an dieser unter mehr oder weniger großem Anpreßdruck und damit reibschlüssig an, so dass obendrein sogar bereits jedes selbsttätige Verrutschen gehemmt ist.

Es hat sich als günstig erwiesen, dass die Länge des elastischen Gurtes einstellbar ist. Bspw. beim Einsatz in einem Fahrzeug kann dabei eine Anpassung an die unterschiedliche Statur verschiedener (Mit-) Fahrer vorgenommen werden. Die Längenverstellung kann bspw. dadurch erfolgen, dass ein variabler Teil des Gurtes jenseits einer verstellbaren Schnalle od. dgl. doppellagig geführt wird.

Gemäß einer bevorzugten Weiterbildung der Erfindung verfügt der Zugkraft-Gurt über eine Einrichtung zum Erfassen der Zugkraft. Damit ist eine Zählung und/oder sonstige Bewertung der von dem Träger bewirkten Bauchmuskel-Kontraktionen möglich. Ggf. könnte die Einrichtung zum Erfassen der Zugkraft auch lösbar angekoppelt sein oder nachrüst- bzw. ankoppelbar.

Als Pendant zu dem/den Verbindungsmittel(n) an einem oder beiden Enden des Zugkraft-Gurtes verfügt auch die Vorrichtung zur Festlegung des Gurtes an der Rückenlehne eines Sitzes über wenigstens ein Verbindungsmittel, bspw. ein Steckverbindungselement, das kompatibel ist zu einem Steckverbindungselement an dem elastischen Gurt.

Die Verbindungsmittel zwischen dem Zugkraft-Gurt einerseits und der Vorrichtung zur Festlegung desselben an der Rückenlehne eines Sitzes andererseits sollten mit Rastelementen versehen sind, die ein selbsttätiges Lösen der Verbindungsmittel verhindern. Es kann sich hierbei um elastisch federnde Zungen an einem Verbindungsmittel handeln, an deren freien Enden je ein oder mehrere, hakenförmige Fortsätze vorgesehen sind, die in entspannt zurückgefedertem Zustand hinter einen dazu komplementären Vorsprung oder sonstigen Fortsatz an dem gegenüberliegenden Verbindungsmittel greifen und sich nur durch manuelles elastisches Weg- bzw. Zurückdrücken der Federzungen lösen lassen.

Die Erfindung erfährt eine vorteilhafte Weiterbildung dadurch, dass an der Vorrichtung zur Festlegung des Zugkraft-Gurtes an der Rückenlehne eines Stuhls, Autositzes, od. dgl. Sitzmöbel eine Lordose vorgesehen ist. Eine solche Lendenwirbelstütze sorgt für eine gerade Körperhaltung und trägt damit zur Schonung der Wirbelsäule bei, was als optimale Vorsorge für eine gute Gesundheit im Alter anzusehen ist.

Die Erfindung empfiehlt, die Lordose zwischen zwei Verbindungsmitteln für den Zugkraft-Gurt anzuordnen. Dadurch sitzt der Zugkraft-Gurt stets symmetrisch vor der Lordose und schafft optimale Voraussetzungen für ein alle Muskelpartien gleichermaßen förderndes Training.

Die Lordose kann als Kissen ausgebildet sein. Hierfür eignet sich bspw. ein mit einem relativ festen Kern versehenes Kissen, aber auch ein aufblasbares Luftkissen, welches über ein Ventil ähnlich einem Schwimmreifen oder Wasserball entweder wie ein Luftballon aufgepustet werden kann oder mittels einer Luftpumpe gefüllt wird, ein Luftkissen hat den Vorteil, dass es bei Personen, welche die Lordose-Funktion nicht wünschen, mit einem schnellen Handgriff entleert und sodann flach und damit unspürbar zusammengelegt werden kann.

Eine besonders einfache Konstruktion ergibt sich, wenn die Vorrichtung zur Festlegung des Zugkraft-Gurtes an einem Sitz dessen Rückenlehne umgreift. Damit ergibt sich ein Formschluß, der - in Verbindung mit der irdischen Schwerkraft - ein selbsttätiges Lösen zuverlässig verhindert. Sofern der Umfang der Befestigungsvorrichtung (in entspanntem Zustand) etwas kleiner dimensioniert ist als der Umfang eines Querschnitts durch die Rückenlehne, so ergibt sich ein weitgehend spielfreier Formschluß.

Da der die Rückenlehne eines Stuhls, Autositzes, od. dgl. umgreifende Gurt über eine rutschhemmende Oberfläche oder Beschichtung an seiner Innenseite verfügt, genügt bereits ein mäßiger Anpreßdruck zur sicheren Fixierung des Befestigungs-Gurtes an einem Sitz.

Im Rahmen einer ersten Ausführungsform der Erfindung ist die Vorrichtung zur Festlegung des Zugkraft-Gurtes an der Rückenlehne eines Stuhls, Autositzes, od. dgl. als in sich geschlossener, über die betreffende Rückenlehne stülpbarer Gurt, Band, Haube od. dgl. ausgebildet. Er könnte solchenfalls sogar als Schutzüberzug für die Rückenlehne dienen. Die Handhabung ist extrem einfach.

Bei einer anderen Ausführungsform ist die Vorrichtung zur Festlegung des Zugkraft-Gurtes an der Rückenlehne eines Stuhls, Autositzes, od. dgl. als in sich schließbarer, um die betreffende Rückenlehne legbarer Gurt, Band od. dgl. ausgebildet. Damit ergibt sich eine Minimalanordnung, die mit wenigen Handgriffen installiert, genauso schnell aber auch wieder entfernt ist.

Der die Rückenlehne eines Stuhls, Autositzes, od. dgl. umgreifende Gurt kann aus mehreren Teilen zusammengesetzt sein, bspw. um einen elastischen Teil mit nicht oder weniger elastischen Segmenten, bspw. im Bereich einer Lordose, zu verbinden.

Die Erfindung zeichnet sich schließlich aus durch zwei Griffelemente mit je einem Verbindungsmittel, bspw. Steckverbindungselement, zum Anschluß an je einem dazu kompatiblen Verbindungsmittel an je einem Ende des Zugkraft-Gurtes. Mit solchen Griffen versehen wird der Zugkraft-Gurt zu einem Expander und kann für vielerlei Übungen der Arme und/oder Beine herangezogen werden.

Weitere Merkmale, Einzelheiten, Vorteile und Wirkungen auf der Basis der Erfindung ergeben sich aus der folgenden Beschreibung einer bevorzugten Ausführungsform der Erfindung sowie anhand der Zeichnung. Hierbei zeigt:
- Fig. 1: ein erfindungsgemäßes Gerät in an der Rückenlehne eines Autositzes befestigtem und von einer Person benutzten Zustand, von der Seite her gesehen;

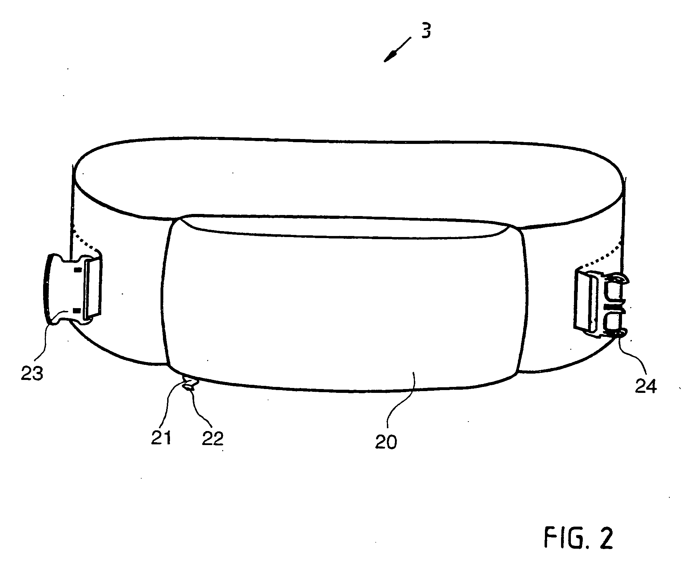

- Fig. 2: die Befestigungsvorrichtung des Geräts aus Fig. 1 in einer Perspektive von vorne;
- Fig. 3: die Befestigungsvorrichtung aus Fig. 2 in an einem Autositz festgelegten Zustand;
- Fig. 4: den eigentlichen Zugkraft-Gurt in einer Ansicht auf eine seiner breiten Seiten;
- Fig. 5: ein Griffstück zur Verbindung mit dem Zugkraft-Gurt aus Fig. 4 in einer Seitenansicht;
- Fig. 6: ein anderes Griffstück zur Verbindung mit dem Zugkraft-Gurt aus Fig. 4, in einer der Fig. 5 entsprechenden Darstellung;
- Fig. 7-9: eine Person bei der Ausführung unterschiedlicher Übungen mit dem durch die Griffstücke nach Fig. 5 und 6 vervollständigten Zugkraft-Gurt aus Fig. 4.

Das erfindungsgemäße Gerät 1 zum Trainieren der Bauchmuskel in sitzender Position umfaßt vier Teile: Einen elastischen, Zugkraft aufnehmenden Gurt 2, einen dessen Festlegung an der Rückenlehne eines Sitzes bewirkenden Gurt 3, sowie zwei Griffelemente 4, 5.

Der Zugkraft-Gurt 2 besteht aus einem elastischen Band 6, welches sich zwischen zwei Verbindungsmitteln 7, 8 erstreckt. Die beiden Verbindungsmittel 7, 8 sind als zueinander komplementäre Steckverbindungselement ausgebildet, so dass der Zugkraft-Gurt 2 auch in sich geschlossen werden könnte, wenngleich dies bei dessen vorgesehener Verwendung nicht beabsichtigt ist.

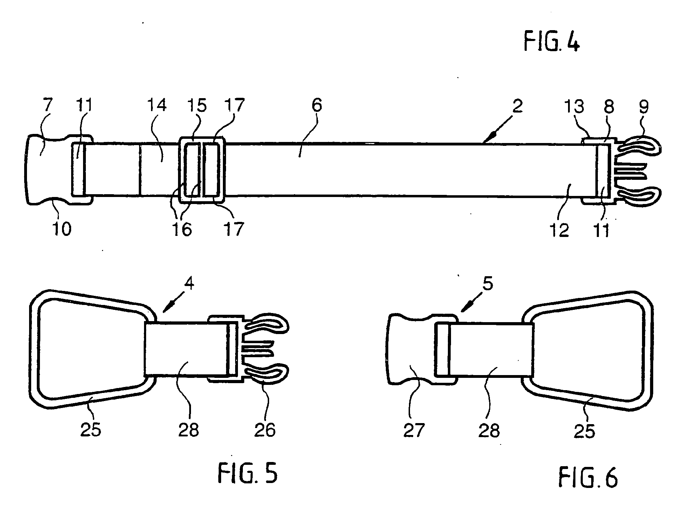

Das in Fig. 4 links dargestellte Steckverbindungselement 7 ist nach Art eines Gurtschlosses aufgebaut mit einer schlitzförmigen Öffnung an seiner dem Zugkraft-Gurt 2 abgewandten Seite zum Einstecken des Steckverbindungselements 8. Dieses umfaßt zwei einander gegenüberliegende Federzungen 9, die elastisch nach innen federn können, wenn sie in das Gurtschloß 7 eingeführt werden, um einen dort vorgesehenen, nach innen vorspringenden Rand an der Mündung der schlitzförmigen Öffnung zu passieren. Jenseits bzw. innerhalb dieses Randes federn sie sodann wieder nach außen und sind dann selbsttätig nicht lösbar. An den Schmalseiten 10 des Gurtschlosses 7 sind jedoch Öffnungen, an denen eine Person die Federzungen 9 nach innen drücken kann, um die Schloßzunge 8 aus dem Gurtschloß 7 herauszuziehen.

Die beiden Steckverbindungselemente 7, 8 verfügen an ihren Rückseiten über je einen Schlitz 11 zum Durchstecken des Zugkraft-Gurtes 2. Ein durchgestecktes Ende 12 des Zugkraft-Gurtes 2 ist sodann um einen den betreffenden Schlitz 11 teilweise begrenzenden Steg 13 geschlungen und bspw. mit sich selbst vernäht.

Vor dem Durchfädeln des gegenüberliegenden Endes 14 des Zugkraft-Gurtes 2 durch den Schlitz 11 an dem anderen Steckverbindungselement 7 ist zunächst eine Art Schnalle 15 auf den Zugkraft-Gurt 2 aufgesteckt. Die Schnalle 15 besteht aus drei zueinander parallelen Stegen 16 sowie aus zwei deren benachbarte Enden jeweils miteinander verbindenden Schenkeln 17. Der Zugkraft-Gurt 2 ist dabei durch die beiden Öffnungen zwischen je zwei Stegen 16 hindurchgefädelt. Das noch freie Ende 14 des Zugkraft-Gurtes wird alsdann durch den Schlitz 11 des zweiten Steckverbindungselements 7 hindurchgesteckt, dann zurückgeführt zu der Schnalle 15 und wird dort jeweils zusätzlich zu dem Mittelstück des Zugkraft-Gurtes 2 nochmals durch beide Schnallenöffnungen gesteckt. Es wird schließlich um dem mittleren Steg 16 herumgeschlungen und mit sich selbst vernäht. Durch Verschieben der Schnalle 15 entlang des Zugkraft-Gurtes 2 kann die Länge desselben variiert und an den Taillenumfang einer beliebigen Person angepaßt werden.

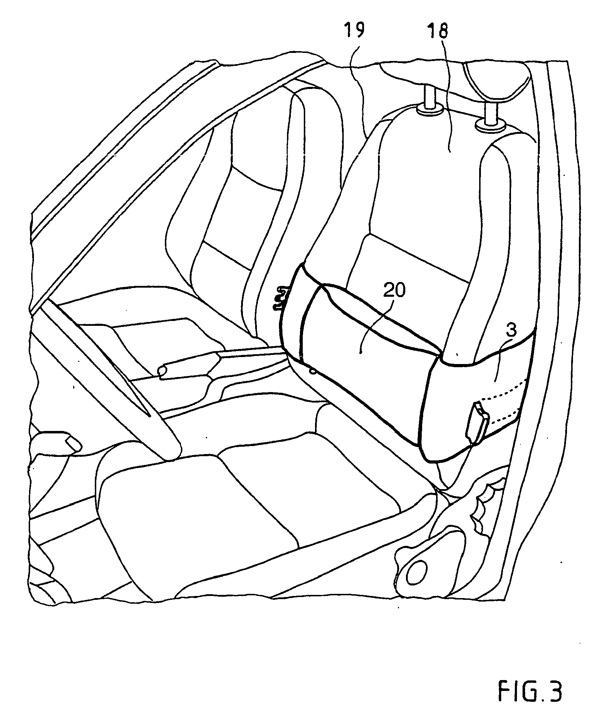

Der Befestigungs-Gurt 3 ist in sich ringförmig geschlossen. Das Material muß nicht elastisch sein; bevorzugt hat es jedoch eine (geringe) Dehnungsfähigkeit, um über die Rückenlehne 18 eines jeden beliebigen Sitzes 19 übergestreift werden zu können, wie Fig. 3 zu entnehmen ist. Weiter bevorzugt ist nur ein Abschnitt des Befestigungs-Gurtes 3, elastisch ausgebildet, der übrige kann inelastisch sein.

Der Befestigungs-Gurt 3 ist mit einer Lordose 20 versehen, die im dargestellten Beispiel als mit Luft befüllbares Kissen ausgebildet ist. Zur Befüllung ist ein Ventil 21 vorgesehen mit einem entfernbaren Stöpsel 22. Das Luftkissen 20 erstreckt sich in aufgeblähtem Zustand nahezu über die gesamte Breite der Rückenlehne 17; die Höhenerstreckung des Kissens 20 entspricht etwa der Breite des Befestigungs-Gurtes 3 oder ist etwas größer als jene. Ein solches Kissen kann an dem vorderen Abschnitt des Befestigungs-Gurtes 3 befestigt sein, es kann aber auch integraler Bestandteil desselben sein. Insofern kann ein solches LordoseKissen 20 ggf. auch die Funktion des elastischen Teils des Befestigungs-Gurtes 3 übernehmen.

Zu beiden Seiten der Lordose 20 ist an dem Befestigungs-Gurt 3 je ein Steckverbindungselement 23, 24 vorgesehen, welches zu je einem Steckvebindungselement 7, 8 des Zugkraft-Gurtes 2 kompatibel ist. Vorzugsweise befindet sich der elastische Bereich des Befestigungs-Gurtes 3 an der Gurtvorderseite zwischen den Steckverbindungselementen 23, 24, während der rückwärtige Abschnitt des Befestigungs-Gurtes 3, insbesondere außerhalb der beiden Steckverbindungselemente 23, 24, nicht elastisch ausgebildet ist und sich daher beim Bauchmuskeltraining weder dehnt noch verschiebt. Die beiden Steckverbindungselemente 23, 24 müssen nicht direkt seitlich an die Lordose 20 anschließen. Vielmehr kann sich dazwischen jeweils ein vorzugsweise elastischer Abschnitt befinden, welche(r) der Anpassung des Gurtumfangs an den Umfang der betreffenden Sitzlehne dient (-en). Die Lordose ist in diesem Fall vergleichsweise schmal und stützt vor allem die Lendenwirbelsäule selbst.

Nachdem eine Person auf dem Fahrzeugsitz 19 gemäß Fig. 3 Platz genommen hat, kann sie daher den Zugkraft-Gurt 2 mit dem Befestigungs-Gurt 3 verbinden und das Bauchmuskeltraining beginnen. Will die betreffende Person kurz aussteigen, bspw. um das Auto zu betanken, so muß nur ein Steckverbindungselement 7, 8 von dem Befestigungs-Gurt 3 gelöst werden; der Zugkraft-Gurt 2 verbleibt dann im Auto und kann nach dem abermaligen Einstieg sofort wieder angelegt und benutzt werden.

Werden dagegen beide Steckverbindungselemente 7, 8 von dem Befestigungs-Gurt 3 gelöst, so können statt dessen die beiden Griffelemente 4, 5 mit je einem Ende 12, 14 des Zugkraft-Gurtes 2 verbunden werden. Die beiden Griffelemente 4, 5 bestehen aus je einem ringförmig in sich geschlossenen Griff 25, bspw. aus einem zu einem trapezförmigen, rechteckigen oder quadratischen Grundriß gebogenen und verschweißten Rundstab, ferner aus je einem Steckverbindungselement 26, 27 zum Zusammenstecken mit je einem Steckverbindungselement 7, 8 des Zugkraft-Gurtes 2, und aus einer kurzen, Griff 25 und Verbindungselement 26, 27 durchgreifenden Schlaufe 28. Auch die Steckverbindungselemente 26, 27 der beiden Griffelemente 4, 5 sind komplementär zueinander.

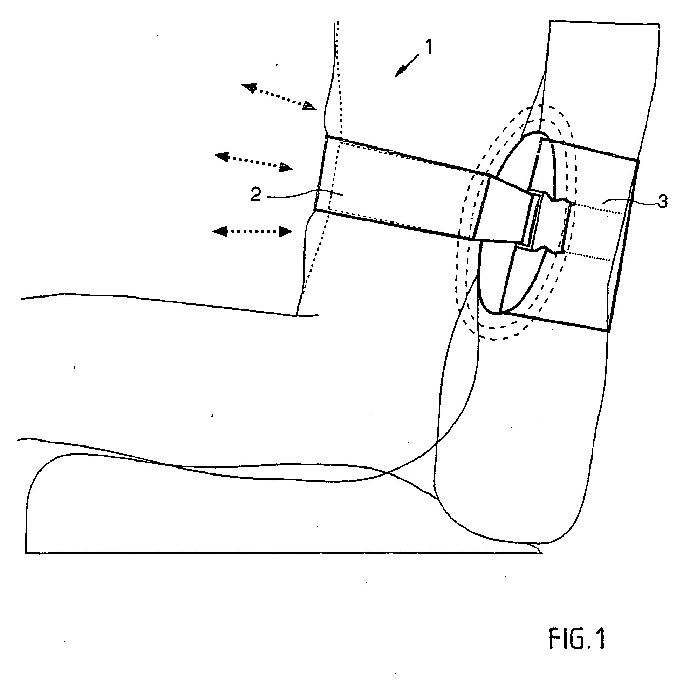

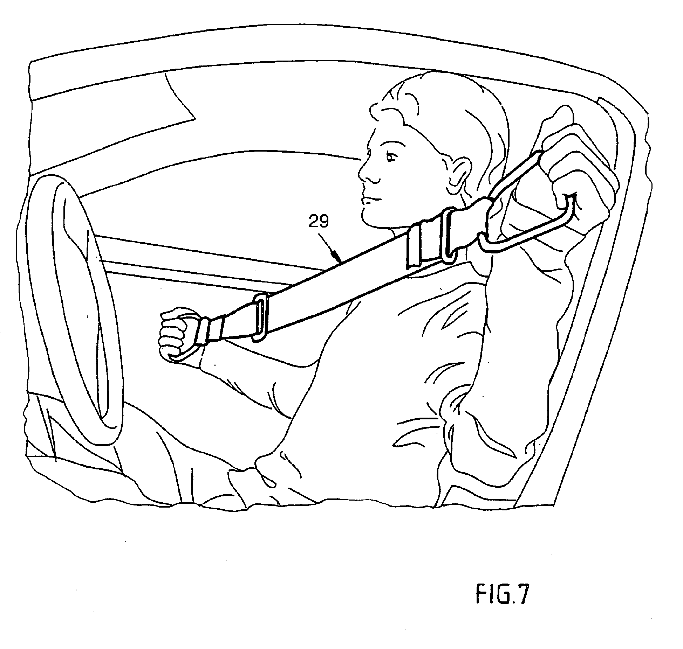

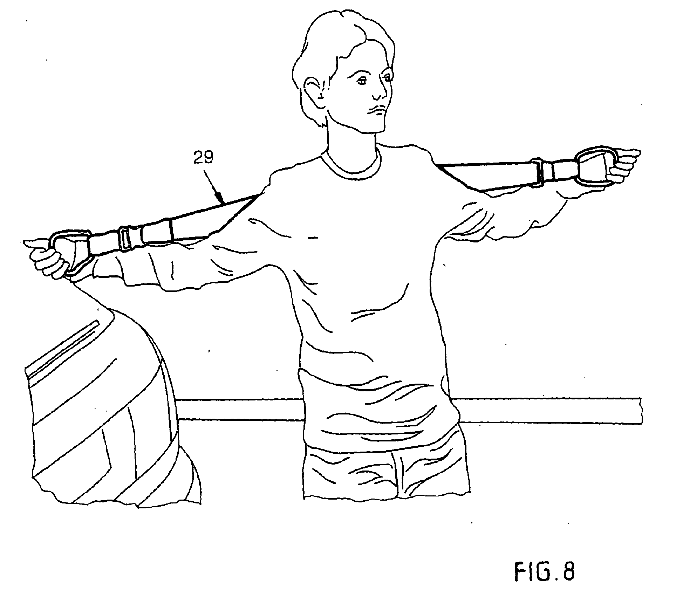

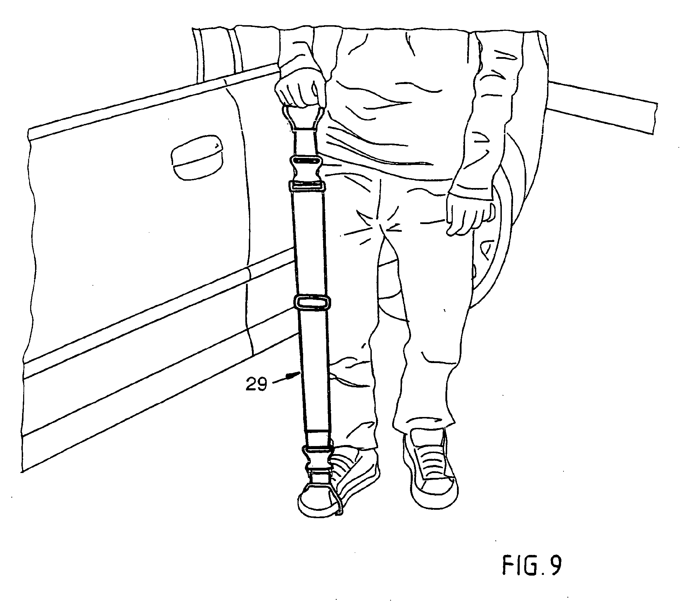

Nach Zusammenstecken des Zugkraft-Gurtes 2 mit den beiden Griffelementen 4, 5 ergibt sich eine Art Expander 29, mit dem verschiedene Übungen durchgeführt werden können, bspw. ein- oder beidseitiges Armstrecken mit dem Expander 29 vor dem Oberkörper (vgl. Fig. 7), beidseitiges Armstrecken mit dem Expander 29 hinter dem Oberkörper (vgl. Fig. 8) oder Anwinkeln des Unterarms entgegen der Zugkraft des mit seinem gegenüberliegenden Ende an einem Fuß verankerten Expanders 29 (vgl. Fig. 9). Während das Bauchmuskeltraining (vgl. Fig. 1) während einer Fahrt ständig geübt werden kann, eignen sich die Expander-Übungen eher für Fahrtpausen, bspw. an einer Raststätte.

## Patentansprüche

1. Gerät (1) zum Trainieren der Bauchmuskel in sitzender Position, mit einem Gurt (2), der vom Anwender durch aktive Muskelanspannung bewegt wird, und mit einer Einrichtung (3) zur vorzugsweise lösbaren Festlegung des Gurtes (2) an der Rückenlehne (18) eines Stuhls, Autositzes (19), oder eines sonstigen Sitzmöbels, wobei der Gurt (2)
a) den Bauch durch Ausübung von Zugkraft einengt;
b) die Taille einer Person nur teilweise umschließt, nämlich auf höchstens 270° oder weniger, während der verbleibende Taillenumfang von der Befestigungseinrichtung (3) umschlossen wird;
c) an seinen beiden Enden (12,14) je ein Mittel zur Verbindung mit der Befestigungseinrichtung (3) aufweist, bspw. ein Steckverbindungselement (7,8); sowie
d) ein sich zwischen den Verbindungsmitteln (7,8) erstreckendes, zumindest bereichsweise elastisches Band (6) aufweist;
und wobei die Befestigungseinrichtung (3)
e) als in sich ringförmig geschlossener oder schließbarer Befestigungsgurt ausgebildet ist und
f) zwei Verbindungsmittel (23,24) für den Zugkraft-Gurt (2) aufweist,
**dadurch gekennzeichnet, dass**
g) die Verbindungsmittel (23,24) den Befestigungsgurt (3) in einen vorderen und einen hinteren Abschnitt unterteilen, wobei sich im vorderen Gurtabschnitt wenigstens ein elastischer Bereich befindet, während der hintere Gurtabschnitt nicht elastisch ausgebildet ist.

2. Gerät nach Anspruch 1, **dadurch gekennzeichnet, dass** die Länge des Zugkraft-Gurtes (2) einstellbar ist.

3. Gerät nach einem der vorhergehenden Ansprüche, **dadurch gekennzeichnet, dass** wenigstens ein Verbindungsmittel (23,24) an der Vorrichtung (3) zur Festlegung des Zugkraft-Gurtes (2) an der Rückenlehne (18) eines Stuhls, Autositzes (19) od. dgl. als Steckverbindungselement (23,24) ausgebildet ist, das kompatibel ist zu einem Steckverbindungselement (7,8) an dem Zugkraft-Gurt (2).

4. Gerät nach einem der vorhergehenden Ansprüche, **dadurch gekennzeichnet, dass** die Verbindungsmittel (7,8;23,24) zwischen dem Zugkraft-Gurt (2) und der Vorrichtung (3) zur Festlegung desselben an der Rückenlehne (18) eines Stuhls, Autositzes (19) od. dgl. mit Rastelementen (9) versehen sind, die ein selbsttätiges Lösen der Verbindungsmittel (7,8;23,24) verhindern.

5. Gerät nach einem der vorhergehenden Ansprüche, **dadurch gekennzeichnet, dass** an der Vorrichtung (3) zur Festlegung des Zugkraft-Gurtes (2) an der Rückenlehne (18) eines Stuhls, Autositzes (19) od. dgl. eine Lordose (20) vorgesehen ist.

6. Gerät nach Anspruch 5, **dadurch gekennzeichnet, dass** sich die Lordose (20) zwischen zwei Verbindungsmitteln (23,24) für den Zugkraft-Gurt (2) befindet.

7. Gerät nach Anspruch 5 oder 6, **dadurch gekennzeichnet, dass** die Lordose (20) als Kissen ausgebildet ist, insbesondere als aufblasbares Luftkissen.

8. Gerät nach einem der vorhergehenden Ansprüche, **dadurch gekennzeichnet, dass** die Vorrichtung (3) zur Festlegung des Zugkraft-Gurtes (2) an einem Stuhl, Autositz (19) od. dgl. dessen Rückenlehne (18) umgreift.

9. Gerät nach Anspruch 8, wobei die Vorrichtung (3) zur Festlegung des Zugkraft-Gurtes (2) an der Rückenlehne (18) eines Stuhls, Autositzes (19) od. dgl. als in sich geschlossenerGurt ausgebildet ist, **dadurch gekennzeichnet, dass** der Befestigungsgurt (3) über die betreffende Rückenlehne (18) stülpbar ausgebildet ist.

10. Gerät nach Anspruch 8, wobei die Vorrichtung (3) zur Festlegung des Zugkraft-Gurtes (2) an der Rückenlehne (18) eines Stuhls, Autositzes (19) od. dgl. als in sich schließbarer Gurt ausgebildet ist, **dadurch gekennzeichnet, dass** der Befestigungsgurt (3) um die betreffende Rückenlehne (18) legbar ausgebildet ist.

11. Gerät nach einem der vorhergehenden Ansprüche, **gekennzeichnet durch** zwei Griffelemente (4,5) mit je einem Verbindungsmittel, bspw. Steckverbindungselement (26,27), zum Anschluß an je einem dazu kompatiblen Verbindungsmittel (7,8) an je einem Ende (12,14) des Zugkraft-Gurtes (2).

## Claims

1. An apparatus (1) for exercising abdominal muscles in a sitting position, comprising a belt (2), which is moved by a user by active muscular exertion, and a fastening means (3) for preferably detachably affixing said tensile belt (2) to a backrest (18) of a selected one of a chair, a car seat (19) and other article of a seating furniture, whereby said tensile belt (2)
a) is adapted to constrain the abdomen by exerting tensile force,
b) encircles the waist of the user only partially, namely over no more than 270° or less, whilst the remainder of the waist is embraced by said fastening means (3),
c) comprises a means for connection to the fastening means (3) at each of its ends (12, 14), for example a connector element (7, 8), and
d) comprises an at least partially elastic strap (6) extending between said two connector elements (7, 8);
and whereby said fastening means (3)
e) is implemented as annular closed or closable affixing belt (3), and
f) comprises two connector elements (23,24) for said tensile belt (2),
**characterized in that**
g) said connector elements (23,24) subdivide said affixing belt (3) into a forward region and a rearward region, whereby the forward region of the belt (3) comprises an elastic area, whilst the rearward region of the belt (3) is not elastic.

2. Apparatus according to claim 1, **characterized in that** the length of said tensile belt (2) is adjustable.

3. Apparatus according to one of the preceding claims, **characterized in that** at least one connecting means disposed on said system (3) for affixing said tensile belt (2) to the backrest (18) of a chair, car seat (19) or the like is formed as a mating connector element (23,24), which is compatible with a mating connector element (7,8) on said tensile belt (2).

4. Apparatus according to one of the preceding claims, **characterized in that** said connecting means (7,8;23,24) between said tensile belt (2) and said system (3) for attaching same to the backrest (18) of a chair, car seat (19) or the like is provided with detent elements (9) that prevent said connecting means (7,8;23,24) from disconnecting on their own.

5. Apparatus according to one of the preceding claims, **characterized in that** a lordosis support (20) is provided on said system (3) for affixing said tensile belt (2) to the backrest (18) of a chair, car seat (19) or the like.

6. Apparatus according to claim 5, **characterized in that** said lordosis support (20) is disposed between two connecting means (23,24) for said tensile belt (2).

7. Apparatus according to claim 5 or 6, **characterized in that** said lordosis support (20) is implemented as a cushion, particularly as an inflatable air cushion.

8. Apparatus according to one of the preceding claims, **characterized in that** said system (3) for affixing said tensile belt (2) to a chair, car seat (19) or the like extends around the backrest (18) thereof.

9. Apparatus according to claim 8, whereby said system (3) for affixing said tensile belt (2) to the backrest (18) of a chair, car seat (19) or the like is implemented as a belt that is fastened to itself, **characterized in that** said affixing belt (3) can be slipped over the backrest (18) concerned.

10. Apparatus according to claim 8, whereby said system (3) for affixing said tensile belt (2) to the backrest (18) of a chair, car seat (19) or the like is implemented as a belt that is able to be fastened to itself, **characterized in that** said affixing belt (3) can be placed around the backrest (18) concerned.

11. Apparatus according to one of the preceding claims, **characterized by** two grip elements (4,5) each comprising a connecting means, for example a mating connector element (26,27), for connection to a respective connecting means (7,8) compatible therewith and disposed at a respective end (12,14) of said tensile belt (2).

## Revendications

1. Appareil (1) pour entraîner les abdominaux en position assise avec une sangle (2) que l'utilisateur fait bouger par tension active des muscles et avec un dispositif (3) servant à la fixation de préférence amovible de la sangle (2) au dossier (18) d'une chaise, d'un siège de voiture (19) ou de tout autre siège, la sangle (2)
a) resserrant le ventre en exerçant une tension ;
b) entourant seulement partiellement la taille d'une personne, à savoir au maximum sur 270° ou moins, tandis que le tour de taille restant est entouré par le dispositif de fixation (3) ;
c) présentant respectivement à chacune de ses deux extrémités (12, 14) un moyen d'assemblage au dispositif de fixation (3), par exemple un élément d'assemblage par emboîtement (7, 8) ;
d) présentant une bande élastique (6), tout au moins en partie, qui s'étend entre les moyens d'assemblage (7, 8) ;
et où le dispositif de fixation (3)
e) est réalisé sous forme de sangle de fixation annulaire fermée ou pouvant être fermée et
f) présente deux moyens d'assemblage (23, 24) pour la sangle de tension (2), **caractérisé en ce que**
g) les moyens d'assemblage (23, 24) subdivisent la sangle de fixation (3) en un segment avant et arrière, où au moins une zone élastique se trouve dans le segment avant de la sangle tandis que le segment arrière de la sangle n'est pas réalisé sous forme élastique.

2. Appareil selon la revendication 1, **caractérisé en ce que** la longueur de la sangle de tension (2) est réglable.

3. Appareil selon l'une des revendications précédentes, **caractérisé en ce qu'**au moins un moyen d'assemblage (23, 24) sur le dispositif (3) de fixation de la sangle de tension (2) sur le dossier (18) d'une chaise, d'un siège de voiture (19) ou similaire est réalisé sous forme d'élément d'assemblage par emboîtement (23, 24), lequel est compatible avec un élément d'assemblage par emboîtement (7, 8) de la sangle de tension (2).

4. Appareil selon l'une des revendications précédentes, **caractérisé en ce que** les moyens d'assemblage (7, 8 ; 23, 24) entre la sangle de tension (2) et le dispositif (3) pour sa fixation sur le dossier (18) d'une chaise, d'un siège de voiture (19) ou sim. sont dotés d'éléments d'enclenchement (9) qui empêchent les moyens d'assemblage (7, 8 ; 23, 24) de se détacher automatiquement.

5. Appareil selon l'une des revendications précédentes, **caractérisé en ce que** l'on prévoit une lordose (20) sur le dispositif (3) de fixation de la sangle de tension (2) sur le dossier (18) d'une chaise, d'un siège de voiture (19) ou similaire.

6. Appareil selon la revendication 5, **caractérisé en ce que** la lordose (20) se trouve entre les deux moyens d'assemblage (23, 24) pour la sangle de tension (2).

7. Appareil selon la revendication 5 ou 6, **caractérisé en ce que** la lordose (20) est réalisée sous forme de coussin, en particulier sous forme de coussin d'air gonflable.

8. Appareil selon l'une des revendications précédentes, **caractérisé en ce que** le dispositif (3) de fixation de la sangle de tension (2) entoure le dossier (18) d'une chaise, d'un siège de voiture (19) ou similaire.

9. Appareil selon la revendication 8, où le dispositif (3) de fixation de la sangle de tension (2) sur le dossier (18) d'une chaise, d'un siège de voiture (19) ou sim. est réalisé sous forme de sangle fermée, **caractérisé en ce que** la sangle de fixation (3) est réalisée de façon à être enfilée sur le dossier en question (18).

10. Appareil selon la revendication 8, où le dispositif (3) de fixation de la sangle de tension (2) sur le dossier (18) d'une chaise, d'un siège de voiture (19) ou sim. est réalisé sous forme de sangle pouvant être fermée, **caractérisé en ce que** la sangle de fixation (3) est réalisée de façon à être mise autour du dossier en question (18).

11. Appareil selon l'une des revendications précédentes, **caractérisé en ce que** deux éléments de préhension (4, 5) avec respectivement un moyen d'assemblage, par ex. un élément d'assemblage par emboîtement (26, 27), pour le raccordement à respectivement un élément d'assemblage (7, 8) compatible avec chacune des extrémités (12, 14) de la sangle de tension (2).
